# EUROPÄISCHE PATENTSCHRIFT

(11) **EP 0 948 784 B1**
(45) Veröffentlichungstag und Bekanntmachung des Hinweises auf die Patenterteilung: **20.03.2002**
(21) Anmeldenummer: 96943034.7
(22) Anmeldetag: 04.12.1996
(51) Int. Cl.: G09B 23/28, A61L 27/00

(54) **KÜNSTLICHES GEWEBE**
ARTIFICAL TISSUE
TISSU ARTIFICIEL

(43) Veröffentlichungstag der Anmeldung: 13.10.1999
(73) Patentinhaber: Erbe Elektromedizin GmbH., D-72072 Tübingen (DE)
(72) Erfinder: GRUND, Karl, Ernst, D-72070 Tübingen (DE); KÖHN, Petra, C., D-72076 Tübingen (DE); FARIN, Günter, D-72070 Tübingen (DE)
(74) Vertreter: Kruspig, Volkmar, Dipl.-Ing.
(86) Internationale Anmeldenummer: EP9605420
(87) Internationale Veröffentlichungsnummer: WO9825254

(56) Entgegenhaltungen:
- DE-A- 19 538 015
- US-A- 4 277 367

## Beschreibung

Die Erfindung betrifft ein künstliches Gewebe sowie künstliche Organe, Organteile oder Organsysteme und eine Verwendung von künstlichen Geweben oder künstlichen Organen zum chirurgischen Operationstraining.

Aus der US 4,277,368 ist ein künstliches Gewebematerial bekannt, das bezüglich der Adsorption von Ultraschallsignalen bestimmte Eigenschaften aufweist. Konkret wird auf eine aus einem Gel, insbesondere Hydrogel bestehende Matrix zurückgegriffen, wobei eine Schallausbreitungsgeschwindigkeit im Bereich von 1400 bis 1650 m/s einzustellen ist. Zum Erhalt der gewünschten Eigenschaften wird beispielsweise Graphitpulver, Talkum oder kugelförmiges Polyethylen zugegeben.

Künstliche Organe, Organteile oder Organsysteme für Unterrichtszwecke im Fach Anatomie sind bereits seit vielen Jahrzehnten bekannt. Es existieren jedoch keine künstlichen Organe, Organteile oder Organsysteme, an welchen spezielle diagnostische und/oder interventionelle, wie z.B. HF-chirurgische Verfahren oder dergleichen trainiert werden können.

Ein Training an künstlichen Organen, Organteilen oder Organsystemen ist aber insbesondere in der starren wie in der flexiblen Endoskopie außerordentlich wichtig, um eine sichere Handhabung der endoskopischen Instrumente zu erlernen.

Bekanntermaßen enthalten operative Verfahren der Endoskopie, wie beispielsweise die endoskopische Polypektomie und die transurethrale Resektion der Prostata, relativ viele variable und interdependente Parameter, so daß es sich zur sicheren Anwendung und zur Vermeidung von Komplikationen bei entsprechenden operativen Verfahren als dringend notwendig erweist, ein umfassendes Training durchzuführen.

Ein wichtiger Parameter bei allen operativen Verfahren, bei denen die Hochfrequenzchirurgie genutzt wird, ist die Hochfrequenzleistung, deren Intensität sowohl beim Koagulieren als auch beim Schneiden entscheidend ist. Das Einstellen der jeweiligen Leistung und das Führen des Operationsinstrumentes erfordern Erfahrung und Geschick, das nur durch entsprechendes Training zu erlernen ist.

Unter Koagulieren wird die Anwendung hochfrequenten elektrischen Wechselstromes zur lokalen endogenen Erwärmung biologischen Gewebes verstanden, wobei die Erwärmung bis zu einer Temperatur erfolgt, bei welcher intra- und extrazelluläre kolloidale Gewebebestandteile aus dem Sol in einen Gelzustand übergehen. Zusätzliche Erwärmung des koagulierten Gewebes führt zu m Austrocknen, d. h. zur Desikkation, wodurch das Gewebevolumen schrumpft. Eine weitere Erwärmung des ausgetrockneten Gewebes führt zur Karbonisation, d. h. zur Verbrennung. Die drei vorgenannten thermisch verursachten Nekrosestadien unterscheiden sich lediglich durch die hierfür erforderliche Wärmemenge.

Bereits hieraus wird deutlich, daß große Erfahrungswerte vorliegen müssen, um die gewünschte Technik erfolgreich anwenden zu können. Beispielsweise reicht in einigen Fällen für eine effiziente Hämostase, d. h. Blutstillung die Koagulation, in anderen die Desikkation aus. Für das Schneiden mittels HF-chirurgischer Verfahren ist es notwendig, Energie dergestalt zuzuführen, daß gezielt und schnell ein endogenes Erwärmen des biologischen Gewebes bis zu einer Temperatur erfolgt, bei welcher intra- und extrazelluläres Wasser so schnell verdampft, daß hierüber die Zellmembranen durch den plötzlichen Dampfdruck zerrissen werden.

Zusätzliche Schwierigkeiten entstehen dann, wenn in der Gastroenterologie unter Zuhilfenahme endoskopischer Instrumentarien mittels Argon-Plasma-Koagulationen durchgeführt werden sollen.

Es ist daher Aufgabe der Erfindung, ein künstliches Gewebe sowie künstliche Organe anzugeben, das bzw. die in effektiver Weise ein realitätsnahes Training und Ausbildung zur Handhabung chirurgischer Operationstechniken ermöglichen.

Die Lösung der Aufgabe der Erfindung erfolgt mit einem Gegenstand nach den Merkmalen der Patentansprüche 1, 11 und/oder 12, wobei die Unteransprüche mindestens zweckmäßige Ausgestaltungen und Weiterbildungen umfassen.

Der Grundgedanke der Erfindung besteht darin, ein künstliches Gewebe anzugeben, welches zum einen eine entsprechende elektrische Leitfähigkeit aufweist, so daß die physikalischen Effekte, die das Schneiden bei der HF-Chirurgie ermöglichen, eintreten, weiterhin Wasser im Gewebe eingebunden ist, welches verdampfen kann und darüber hinaus Mittel vorgesehen sind, welche festen organischen Gewebebestandteilen gleichkommen, so daß eine Verbrennung selbiger analog dem natürlichen Gewebe möglich ist.

Darüber hinaus ist, dem Grundgedanken der Erfindung folgend, das künstliche Gewebe formbar, so daß natürliche Organe, Organteile oder Organsysteme nachgebildet werden können.

So wird erfindungsgemäß vorgeschlagen, ausgehend von formbeständigen Hydrogelen für das künstliche Gewebe zusätzlich der Mischung zur Bildung des Gewebes einen Elektrolyten sowie brennbare Fasern, beispielsweise Baumwolle oder dergleichen, beizugeben.

Dadurch, daß gemäß einem weiteren Grundgedanken der Erfindung unterschiedliche Mischungsverhältnisse der oben genannten Bestandteile realisiert werden können und innerhalb einer Gewebestruktur verschiedene künstliche Gewebe aus unterschiedlichen Mischungsverhältnissen miteinander verbunden sein können, sind natürliche Gewebe mit unterschiedlichen Strukturen oder Strukturverteilungen nachbildbar.

In einer Ausführungsform werden dem künstlichen Gewebe Farbstoffe zugesetzt, so daß ein dem natürlichen Gewebe entsprechendes Aussehen einstellbar ist. Vorteilhafterweise werden Farbstoffe verwendet, welche bei einer oder mehreren verschiedenen definierten Temperaturen, beispielsweise bei der Temperatur, bei welcher natürliche, menschliche Gewebe thermisch koagulieren, einen oder mehrere Farbumschläge zeigen. Vorteilhafterweise werden Thermocolore eingesetzt, die im Temperaturbereich von ca. 50 und 100 °C einen Farbumschlag zeigen. Hierdurch ist es möglich, die Temperaturentwicklung im künstlichen Gewebe während und nach einem Schneide- und/oder Koagulationsvorgang zu beobachten und zu kontrollieren.

In dem Falle, wo künstliche Gewebe verwendet werden, welche einen oder mehrere temperaturabhängige Farbumschläge zeigen, sind diese einerseits zum Trainieren von Schneideffekten, andererseits aber auch zum Trainieren von thermischen Koagulationstechniken, wie beispielsweise monopolarer oder bipolarer Kontaktkoagulation, Argon-Plasma-Koagulation und Laser-Koagulation, einsetzbar.

Erfindungsgemäß wird gemäß einem weiteren Grundgedanken der Erfindung der Mischung zur Bildung künstlicher Gewebe ein hygroskopisches Mittel, beispielsweise Glyzerin, beigegeben, so daß ein frühzeitiges, unerwünschtes Austrocknen verhindert wird. Zusätzlich verbessert die GlyzerinBeimischung die Verbrennung der im Gewebe enthaltenen Fasern. Ebenso ist eine Beimischung von aus der Lebensmittelchemie bekannten Konservierungsmitteln zur Vermeidung von Schimmelbildung vorteilhaft.

Erfindungsgemäß sind aus den künstlichen Geweben Organe, Organteile oder Organsysteme formbar, die nicht nur der Darstellung der Anatomie sowie pathologische Veränderungen dienen, sondern die auch zum Training chirurgischer Interventionen verwendet werden können.

Hierdurch wird der Chirurg in die Lage versetzt, zum einen die jeweiligen pathologischen Veränderungen, z. B. Polypen, Tumore, Ulzera usw. zu erkennen, und zum anderen darüber hinaus durch eine entsprechende Operationsmethode, z. B. Schneiden, Koagulieren, Vaporisieren, Argon-Plasma-Koagulieren, Laser-Applizieren, derartige krankhafte Veränderungen im Training chirurgisch zu behandeln oder zu entfernen.

Eine spezielle Ausführungsform der Erfindung geht davon aus, daß in den künstlichen Geweben Gefäßstrukturen und Körperhöhlen eingeformt werden können, die mit Röntgenkontrastmitteln, Konkrementen, Flüssigkeiten oder Gasen füllbar sind, um diagnostische Verfahren, wie beispielsweise bildgebende Verfahren sowie kombinierte Verfahren, wie beispielsweise die Papillotomie oder die Steinextraktion, zu trainieren. Derartige künstliche Körperhöhlen werden auch zum Training endoskopischer HF-chirurgischer Verfahren verwendet.

Alles in allem gelingt es mit der Erfindung ein künstliches Gewebe bereitzustellen, welches insbesondere beim Schneiden oder Koagulieren mittels HF-Strom sowie Laser-Applikation Effekte zeigt, die denjenigen in natürlichen menschlichen Geweben weitestgehend entsprechen.

So wird infolge der elektrischen Leitfähigkeit die Möglichkeit gegeben, daß HF-Strom fließen kann, wodurch endogene Wärme entsteht. Hierdurch wiederum wird die Dampfbildung und folglich Desikkation erreicht. Da die eingesetzten Hydrogele bei höheren Temperaturen vom Gel- in den Solzustand übergehen, d.h. schmelzen und andererseits die beigemischten brennbaren Fasern erst dann durchtrennt werden können, wenn die HF-Spannung ausreichend hoch ist, so daß sich ein elektrischer Lichtbogen zwischen Schneidelektrode und Gewebe ausbildet, werden Effekte erzielt, die sich am Verhalten fester organischer Gewebebestandteile orientieren.

Die Erfindung soll nachstehend anhand eines Ausführungsbeispieles näher erläutert werden.

Ein künstliches Gewebe, aus welchem natürliche Organe, Organteile oder Organsysteme geformt wurden, besteht aus einem wasserhaltigen Material, insbesondere einem Hydrogel, welchem ein Elektrolyt, beispielsweise Natriumchlorid, Kaliumchlorid oder dergleichen, beigemischt wurde. Zusätzlich enthält die Mischung, aus welcher das künstliche Gewebe hergestellt wird, brennbare Fasern, beispielsweise Baumwolle, Leinen oder Kämmling, welcher bei der Schafwollproduktion anfällt.

Als formbeständiges Hydrogel wird beispielsweise eine Mischung aus 4% Agar-Agar und 96% Wasser (93,5% Wasser und 2,5% Glyzerin) oder 20,8% Gelatine und 75,2% Wasser verwendet.

Analog sind auch andere Gele, d. h. an Flüssigkeiten und Gasen reiche disperse Systeme aus mindestens zwei Komponenten verwendbar, die einen festen, kolloid verteilten Stoff und Wasser als Dispersionsmittel aufweisen. Vorteilhaft ist die Verwendung des erwähnten Agar-Agars, eines gelbildenden Heteropolysacchariden, dessen Herstellung kommerziell betrieben wird. Agar-Agar bildet noch in 1%-iger Lösung ein festes Gel, das zwischen 80 und 100 °C schmilzt, und deshalb vorteilhaft angewendet werden kann. Die verwendeten Hydrogele vereinen auf der einen Seite hydrophile, auf der anderen Seite aber wasserunlösliche Eigenschaften und gewährleisten die gewünschte Formerhaltung. Der der Mischung beigefügte Elektrolyt dient dem Erhalt der gewünschten elektrischen Leitfähigkeit und die vorhandenen Fasern bilden dem Natürlichen entsprechend einen mechanischen Widerstand beim Schneiden des Gewebes, welcher nur überwunden werden kann, wenn die Energie, z. B. die HF-Spannung so hoch ist, daß sich ein Lichtbogen zwischen Schneidelektrode und Gewebe ausbildet und die im Lichtbogen befindliche Faser verbrennen.

Die durch den Elektrolyt gegebene elektrische Leitfähigkeit ermöglicht das Fließen von HF-Strom im künstlichen Gewebe, wodurch endogene Wärme entsteht. Die Wärme führt zur Dampfbildung und Desikkation des künstlichen Gewebes. Wie erwähnt, werden brennbare Fasern dem künstlichen Gewebe beigefügt, um einen Schneideffekt zu erreichen, der der HF-Chirurgie im natürlichen menschlichen Gewebe entspricht, bei welchem feste organische Gewebebestandteile ein kraftloses Hindurchschmelzen der Schneidelektrode verhindern. Mit anderen Worten führt die Beimischung brennbarer Fasern, beispielsweise Baumwolle oder dergleichen in das Hydrogel zur Nachbildung der Eigenschaften natürlicher, menschlicher Gewebe, so daß das künstliche Gewebe entsprechend der genannten Zusammensetzung bezüglich des Schneideffektes mit HF-Strom ähnliche Eigenschaften zeigt.

In einem weiteren Ausführungsbeispiel wird dem künstlichen Gewebe mit der erwähnten Zusammensetzung ein Farbstoff beigegeben, so daß sich ein entsprechendes natürliches Aussehen einstellt. Besonders vorteilhaft ist die Verwendung von Farbstoffen, bei welchen Farbumschläge in vorgegebenen Temperaturbereichen auftreten. Vorteilhaft ist die Anwendung sogenannter Thermocolore, die ein geändertes Farbverhalten im Temperaturbereich von ca. 50 und 100°C zeigen. Bei Anwendung derartiger Farbstoffe kann die Temperaturentwicklung im künstlichen Gewebe während und nach einem Schneide- und/oder Koagulationsvorgang beobachtet und kontrolliert werden, wodurch sich der Ausbildungs- und Lerneffekt verbessert.

In dem Falle, wo künstliche Gewebe eingesetzt werden, welche einen oder mehrere temperaturabhängige Farbumschläge zeigen, eignen sich diese nicht nur zum Trainieren von Schneideffekten, sondern auch zum Training von thermischen Koagulationstechniken, wie beispielsweise der monopolaren oder bipolaren Kontakt-Koagulation, Argon-Plasma-Koagulation oder der Laser-Koagulation.

Bei einem weiteren Ausführungsbeispiel wird eine künstliche, unterschiedliche Gewebestruktur dadurch nachgebildet, indem einzelne Gewebebestandteile unterschiedliche Mischungsverhältnisse zwischen Hydrogel und Faseranteil sowie Elektrolyten aufweisen, so daß unterschiedliche physikalische Eigenschaften simulierbar sind. Zusätzlich können den unterschiedlich gemischten, künstlichen Geweben unterschiedliche Farben beigegeben werden, um die Gewebestrukturen auch visuell deutlich zu machen.

Es hat sich gezeigt, daß das Beimischen hygroskopischer Mittel, beispielsweise Glyzerin oder dergleichen, ein frühzeitiges Austrocknen des künstlichen Gewebes verhindert, so daß dessen Lager- und Einsatzfähigkeit über einen längeren Zeitraum gewährleistet ist. Zusätzlich verbessert die Glyzerinbeimischung während des Schneidvorganges die Verbrennung der Fasern. Als Konservierungsstoff kann beispielsweise 0,1% Sorbinsäure (trans-trans-Hexadien-2,4-Säure) oder 0,3% PHB-Ester-Gemisch (0,21% p-Hydroxybenzoesäuremethylester und 0,09% p-Hydroxybenzoesäurepropylester) zugegeben werden.

Durch die Formbarkeit der Mischung können gemäß einem weiteren Ausführungsbeispiel künstliche Organe, Organteile oder Organsysteme mit anatomischen und/oder pathologischen Abnormalitäten bzw. Befunden nachgebildet werden, so daß entsprechende chirurgische Interventionen, insbesondere auch endoskopische Techniken trainiert werden können. Insbesondere zum Training endoskopischer Operationstechniken können die künstlichen Organe, Organteile oder Organsysteme eingeformte Körperhöhlen aufweisen, so daß einerseits diagnostische Verfahren und andererseits aber, wie bereits erläutert, chirurgische Operationstechniken erprobt und erlernt werden können. Es können aber auch pathologische Gewebestrukturen, wie beispielsweise Polypen und/oder Tumore, aus diesem erfindungsgemäßen künstlichen Gewebe geformt werden, welche in ebenfalls künstliche Organe, Organteile oder Organsysteme einfügbar sind.

Die künstlichen Gewebe, Organe, Organteile oder Organsysteme gemäß vorliegenden Ausführungsbeispielen gewährleisten die insbesondere für die HF-Chirurgie erforderlichen Effekte, nämlich zum einen eine ausreichende elektrische Leitfähigkeit, die Verdampfung von enthaltenem Wasser bei HF-Behandlung sowie die Verbrennung fester, organischer Bestandteile dann, wenn sich beispielsweise ein elektrischer Lichtbogen zwischen Schneidelektrode und dem Gewebe ausbildet. Das erfindungsgemäße Gewebe kann außerdem auch zum Erlernen anderer thermischer Operationsverfahren, wie beispielsweise mit Laser, Kautern oder Mikrowelle angewendet werden, wobei jedoch die Beimischung von Elektrolyten zur Nachbildung der elektrischen Leitfähigkeit nicht erforderlich ist.

Es liegt im Sinne der Erfindung, daß das künstliche Gewebe knochenähnliche Stützstrukturen aufweisen kann, so daß sich die Möglichkeit zum Erlernen und Trainieren der Operationstechniken weiter verbessert.

## Patentansprüche

1. Künstliches Gewebe zum chirurgischen Operationstraining, bestehend aus einer formbaren Mischung aus einem Hydrogel,
**dadurch gekennzeichnet,**
**daß** die Mischung weiterhin einen Elektrolyten sowie brennbare Fasern enthält.

2. Gewebe nach Anspruch 1,
**dadurch gekennzeichnet,**
**daß** das Hydrogel Agar-Agar und Wasser enthält.

3. Gewebe nach Anspruch 1,
**dadurch gekennzeichnet,**
**daß** das Hydrogel Gelatine und Wasser enthält.

4. Gewebe nach Anspruch 1
**dadurch gekennzeichnet,**
**daß** das Hydrogel ein hydrophiles, wasserunlösliches Polymer ist.

5. Gewebe nach einem der vorangegangenen Ansprüche,
**dadurch gekennzeichnet,**
**daß** der Elektrolyt Natriumchlorid, Kaliumchlorid oder Kalziumchlorid ist.

6. Gewebe nach einem der vorangegangenen Ansprüche,
**dadurch gekennzeichnet,**
**daß** die brennbaren Fasern aus Baumwolle, Leinen oder Kämmling bestehen.

7. Gewebe nach einem der vorangegangenen Ansprüche,
**gekennzeichnet durch**
Beimischung von Farbstoffe zur farblichen Nachbildung natürlichen Gewebes.

8. Gewebe nach Anspruch 7,
**dadurch gekennzeichnet,**
**daß** die Farbstoffe im Temperaturbereich zwischen 50 und 100°C einen oder mehrere Farbumschläge aufweisen.

9. Gewebe nach einem der vorangegangenen Ansprüche,
**gekennzeichnet durch**
unterschiedliche Mischungsverhältnisse und Zusammenfügen von mehreren Geweben unterschiedlicher Mischungsverhältnisse zur Bildung einer Gewebestruktur.

10. Gewebe nach einem der vorangegangenen Ansprüche,
**gekennzeichnet durch**
Beimischung eines hygroskopischen Mittels, insbesondere Glyzerin zum Haltbarmachen.

11. Künstliches Organ, Organteil oder Organsystem mindestens bestehend aus einem Gewebe nach einem der Ansprüche 1 bis 10.

12. Verwendung von künstlichem Gewebe oder künstlichen Organen, Organteilen oder Organsystemen nach einem der Ansprüche 1 bis 11 zum chirurgischen Operationstraining, insbesondere für das Training HF-chirurgischer Effekte, thermischer Koagulationstechniken, der Argon-Plasma-Koagulation, von Laser-Applikationen, Schneiden, Vaporisieren, Kryo-Behandlungen sowie zur endoskopischen Polypektomie und Papillotomie.

## Claims

1. An artificial tissue for surgical operation training comprising a shapeable mixture of a hydrogel,
**characterized in that**
the mixture further contains an electrolyte and inflammable fibres.

2. Tissue according to claim 1,
**characterized in that**
said hydrogel contains agar-agar and water.

3. Tissue according to claim 1,
**characterized in that**
said hydrogel contains gelatin and water.

4. Tissue according to claim 1,
**characterized in that**
said hydrogel is a hydrophilic water-insoluble polymer.

5. Tissue according to any one of the preceding claims,
**characterized in that**
said electrolyte is sodium chloride, potassium chloride or calcium chloride.

6. Tissue according to any one of the preceding claims,
**characterized in that**
said inflammable fibres consist of cotton, linen or noil.

7. Tissue according to any one of the preceding claims,
**characterized by**
an admixture of colorants for the chromatic reproduction of the natural tissue.

8. Tissue according to claim 7,
**characterized in that**
said colorants have one or more changes of color in the temperature range between 50 and 100°C.

9. Tissue according to any one of the preceding claims,
**characterized by**
differing mixing ratios and combining several tissues of differing mixing ratios for forming a tissue structure.

10. Tissue according to any one of the preceding claims,
**characterized by**
an admixture of a hygroscopic substance, in particular glycerine, for conservation purposes.

11. An artificial organ, organ part or organ system, at least consisting of a tissue according to any one of claims 1 through 10.

12. Use of artificial tissue or artificial organs, organ parts or organ systems according to any one of claims 1 through 11 for surgical operation trainings, in particular for the training of HF-surgical effects, thermal coagulation techniques, the argon-plasma coagulation, of laser applications, cutting, vaporizing, cryo-treatments, as well as for the endoscopic polypectomy and papillotomy.

## Revendications

1. Tissu artificiel pour l'entraînement chirurgical d'opérations, ce tissu comprenant un mélange plastique d'un hydrogel
**caractérisé en ce que**
ledit mélange contient en outre un électrolyte et de fibres inflammables.

2. Tissu selon la revendication 1,
**caractérisé en ce que**
ledit hydrogel contient de l'agar-agar et de l'eau.

3. Tissu selon la revendication 1,
**caractérisé en ce que**
ledit hydrogel contient de la gélatine et de l'eau.

4. Tissu selon la revendication 1,
**caractérisé en ce que**
ledit hydrogel est un polymère hydrophile et insoluble dans l'eau.

5. Tissu selon l'une des revendications précédentes,
**caractérisé en ce que**
ledit électrolyte est du chlorure de sodium, du chlorure de potassium ou du chlorure de calcium.

6. Tissu selon l'une des revendications précédentes,
**caractérisé en ce que**
les fibres inflammables consistent de coton, de lin ou de blousse.

7. Tissu selon l'une des revendications précédentes,
**caractérisé par**
l'addition de colorants pour la reproduction chromatique du tissu naturel.

8. Tissu selon la revendication 1,
**caractérisé en ce que**
lesdits colorants subissent un ou plusieurs changements chromatiques dans une rangée de température entre 50 et 100°C.

9. Tissu selon l'une des revendications précédentes,
**caractérisé par**
différentes proportions de mélange et le rassemblement de plusieurs tissus de différentes proportions de mélange pour la formation d'une structure de tissu.

10. Tissu selon l'une des revendications précédentes,
**caractérisé par**
l'addition d'une substance hygroscopique, en particulier la glycérine, à des fins de conservation.

11. Organe artificiel, partie d'un organe artificiel ou système d'organes artificiels consistant au moins d'un tissu selon l'une des revendications 1 à 10.

12. Emploi du tissu artificiel ou d'organes, parties d'organes artificiels ou systèmes d'organes artificiels selon l'une des revendications 1 à 11 pour l'entraînement chirurgical d'opérations, en particulier pour l'entraînement sur les effets H.F. chirurgicaux, les techniques thermiques de coagulation, la coagulation argon plasma, les applications laser, les coupes chirurgicales, la vaporisation, les cryothérapies, ainsi que pour la polypectomie et papillotomie endoscopique.
